# EUROPEAN PATENT SPECIFICATION

(11) **EP 2 460 509 B1**
(45) Date of publication and mention of the grant of the patent: **15.11.2017**
(21) Application number: 11191792.8
(22) Date of filing: 02.12.2011
(51) Int. Cl.: A61K 8/36, A61K 9/08, A61K 31/19, A61K 9/00, A61P 17/12

(54) **Composition for the treatment of warts**
Zusammensetzung zur Behandlung von Warzen
Composition pour le traitement des verrues

(30) Priority: 06.12.2010 DK 201070530
(43) Date of publication of application: 06.06.2012
(73) Proprietor: ABBEX AB, 170 09 Solna (SE)
(72) Inventor: Licht, Flemming, 2720 Vanløse (DK)
(74) Representative: FRKelly

(56) References cited:
- WO-A1-00/45808
- WO-A2-2008/128627
- US-A- 5 476 664
- US-A- 5 525 358
- SHAMSADINI S ET AL: "Treatment of warts with topical formic acid [1]", IRANIAN JOURNAL OF MEDICAL SCIENCES, SHIRAZ UNIVERSITY PUBLICATIONS, SHIRAZ, IR, vol. 30, no. 4, 1 January 2005 (2005-01-01), page 199, XP009156548, ISSN: 0253-0716
- BHAT R M ET AL: "TOPICAL FORMIC ACID PUNCTURE TECHNIQUE FOR THE TREATMENT OF COMMON WARTS", INTERNATIONAL JOURNAL OF DERMATOLOGY, WILEY-BLACKWELL PUBLISHING LTD, UK, vol. 40, no. 6, 1 June 2001 (2001-06-01), pages 415-419, XP001097776, ISSN: 0011-9059, DOI: 10.1046/J.1365-4362.2001.01242.X
- ABBOTT L G: "WARTS", BULLETIN OF THE POST-GRADUATE COMMITTEE IN MEDICINE, UNIVERSITY OF SYDNEY,, vol. 19, 1 March 1964 (1964-03-01), pages 301-304, XP009156607, ISSN: 0042-0115

## Description

The present invention relates to a composition for use in a method for the treatment of warts. In particular, it concerns a composition for use in a method for the topical treatment of warts in mammals, such as humans.

### Technical Background

The European patent 1143966 B1 describes the use of a preparation comprising formic acid as an active ingredient for the manufacture of a medicament for the treatment of warts caused by virus in a mammal, by topical administration of the medicament on the affected area. The patent teaches that the wart should be softened with hot water and that, if necessary, hard skin covering the wart should be scraped of, before treatment with formic acid.

Bhat et al. ("Topical formic acid puncture technique for the treatment of common warts", International Journal of Dermatology 2001, 40, 415-419) discloses a topical application/needle puncture technique for the treatment of warts.

### Summary of the invention

The needle puncture technique has been regarded as the best wart treatment using formic acid, as the puncture technique has hitherto provided the best reproducible results.

There is a continued need for alternative means for the treatment of warts. Several demands may be put on such means. It may preferably be inexpensive, easy to manufacture, storage stable, effective, and should work shortly after application, allowing warts to be eradicated within a short time span. In addition, it should preferably be easy to apply, non-toxic, and not be associated with discomfort for the patient, i.e. it should not have a repulsive smell or be painful for the patient during or after administration.

Some patients complain that acid based means for the topical treatment of warts have low efficacy, take several weeks to act, are foul-smelling and may be painful to apply.

There is a need for a composition which a patient may apply without the need of any preparatory steps, before application of the composition on the warts.

It has surprisingly been discovered that a composition comprising formic acid and at least one C1-C4 alkyl ester of lactic acid, malic acid, tartaric acid, or citric acid, preferably ethyl lactate, has particular advantages for the topical treatment of warts.

In particular, it has surprisingly been discovered that the inclusion in the composition of at least one C1-C4 alkyl ester of lactic acid, malic acid, tartaric acid, or citric acid, preferably ethyl lactate, provides the same effect as using the puncture technique for improving the effect of the acid on the warts.

It has been discovered that it may not be necessary to remove hard skin covering warts by use of a composition according to the present invention.

Further, it has surprisingly been discovered that the high concentrations proposed by the prior art may not be necessary in all cases, by using a composition according to the present invention.

Preliminary experiments indicate that the inclusion in the composition of at least one C1-C4 alkyl ester of lactic acid, malic acid, tartaric acid, or citric acid, preferably ethyl lactate, reduces the contact angle of the composition, i.e. reduces the surface tension. Without being bound by any theory, it may be speculated that the number or strengths of hydrogen bonds in the composition is decreased by the inclusion of one of the alkyl esters, such as ethyl lactate, thereby decreasing the contact angle. Inclusion of water and/or alcohol may increase the contact angle. Decreased contact angle makes it easier for the composition to adhere to warts, and following to be absorbed by warts, i.e. improving the penetration of the composition of the invention. Improved penetration may further emphasize any antiviral effects of formic acid. Additionally, reduced surface tension makes it easier to apply the composition in the adequate amount, with reduced risk of inadvertently smearing composition on adjacent, healthy skin. The reduced surface tension may easily be tested by placing a drop of the composition of the invention on a plate, and comparing with a drop of a composition of the prior art, such as a composition comprising water and formic acid.

It has been realized by the inventor that it may be beneficial to distinguish between soft warts and hard warts.

In particular, it has been discovered that a composition with a lower contents of acid may be preferable in the treatment of soft warts, as a composition with higher contents of acid is more likely to give damages upon accidental application on the surrounding skin. Further, it has been discovered that the use of a composition with high contents of acid on soft warts gave unwanted side effects in some cases. However, higher contents of acids are particularly suitable for hard wards, which are difficult to remove.

The exact mechanism of action of formic acid in relation to the treatment of warts is not known. Dehydration with subsequent destruction of wart infected tissue has been proposed. In addition, formic acid may prevent virus particles from attaching to healthy cells, thereby inhibiting viral transmission.

It may be speculated that there is a synergistic effect between formic acid and at least one C1-C4 alkyl ester of lactic acid, malic acid, tartaric acid, or citric acid, such as ethyl lactate, providing a strong effect against warts. Without being bound by any theory, the inventor of the present invention speculate that formic acid in itself acts as an active ingredient, while e.g. ethyl lactate acts as a carrier, allowing the concentration of acid to be kept locally sufficiently low to impede irritation for the patient. It is additionally speculated that such a carrier allow the acid to penetrate deeper into the warts. Further, the inventor speculates that formic acid and ethyl lactate in the presence of water may react to form lactic acid, which independently acts as an active ingredient.

Aspects and embodiments of the present invention are provided here. It will be clear for the person skilled in the art that these may be combined.

According to an aspect, the invention concerns the use of formic acid and at least one C1-C4 alkyl ester of lactic acid for the manufacture of a composition for the topical treatment of warts, wherein the total amount (w/w) of said ester in the composition is 10 - 70%.

A composition comprising formic acid and at least one C1-C4 alkyl ester of lactic acid, malic acid, tartaric acid, or citric acid, preferably ethyl lactate, may be used for the topical treatment of warts.

According to an aspect, the invention concerns a composition comprising formic acid and at least one C1-C4 alkyl ester of lactic acid, for use in a method for topical treatment of warts wherein the total amount (w/w) of said ester, in the composition is 10 - 70%.

A pharmaceutical composition for the topical treatment of warts may comprise formic acid and be free of water and/or alcohol.

According to yet an aspect, the invention concerns a pen, comprising a composition for use according to the invention, for topical administration of a composition according to the invention. A pen has shown to be particularly useful for the application of a pharmaceutical composition for use according to the invention, as exact control of the precise location of application of the composition may be achieved by the topical application on a wart with the pen. Further, the composition may be rubbed or massaged into a wart with the tip of the pen.

### Detailed Disclosure

According to an aspect, the invention concerns a composition comprising formic acid and at least one C1-C4 alkyl ester of lactic acid, for use in a method for topical treatment of warts wherein the total amount (w/w) of said ester, in the composition is 10 - 70%.

Such a composition may be applied with a soft cotton pad or cotton stick swab, or a pen, i.e. without scraping skin of the warts and without the need of puncturing the warts before application. Scraping skin of the warts and/or puncturing the warts may be associated with pain and should thus be avoided. Avoiding scraping and/or puncturing may improve patient compliance.

Ethyl lactate, also known as lactic acid ethyl ester, is found naturally, and carries a pleasant odor. Ethyl lactate may be produced from biological sources, e.g. by fermentation. Ethyl lactate may be either the levo (S) or dextro (R) form or a mixture of the two. Industrially produced ethyl lactate may consist of a racemic mixture of levo and dextro forms.

According to an embodiment, ethyl lactate used in the present invention is a racemic mixture.

According to another embodiment, ethyl lactate used in the present invention is ethyl(-)-L-lactate, or more than 50% of the ethyl lactate is ethyl(-)-L-lactate. This may be obtained by using ethyl lactate derived from natural sources. According to a preferred embodiment ethyl lactate is ethylS(-)-2-hydroxy propanoate. Preferably it is obtained by fermentation from sugar.

Compositions for use according to the present invention preferably comprise ethyl lactate. According to an embodiment it is contemplated that this ingredient may be partly or fullysubstituted with at least one C1-C4 alkyl ester of lactic acid, malic acid, tartaric acid, citric acid, or a mixture of any of these. The inventor of the present invention speculates that these alkyl esters may have anti-viral effect and/or suitable transport enhancing properties. Among the C1-C4 of relevance for the present invention may be mentioned methyl, ethyl, n-propyl, isopropyl, n-butyl, sec-butyl, isobutyl and tert-butyl esters. If suitable, any or all carboxy groups may be esterified. Monoalkyl, dialkyl and/or trialkyl esters may be suitable. Preferred esters are ethyl esters, further preferred are isopropyl esters. In addition to ethyl lactate, further preferred compounds are diethyl malate, diisopropyl malate, monoethyl tartrate, diethyl tartrate, monoethyl citrate and triethyl citrate.

According to an embodiment it is further contemplated that ethyl lactate may be partly or fully substituted with other ingredients with similar properties, such as lactic acid, preferably L-lactate, malic acid, tartaric acid, citric acid, acetic acid, glycolic acid, propionic acid, 3-hydroxypropanoic acid, malonic acid, butyric acid, hydroxybutyric acid, 1-propanol, 2-propanol, propionaldehyde, acrolein or sodium lactate, or a mixture of any of these.

A range of types of warts has been identified, varying in shape and site affected, as well as the type of human papilloma virus involved. These include, but are not limited to: Common wart (Verruca vulgaris), flat wart (Verruca plana), filiform or digitate wart, Plantar wart (Verruca Pedis), Mosaic wart, Genital wart (Veneral wart, Condyloma acuminatum, Verruca acuminate), and Periungal wart.

According to an embodiment, the invention concerns a composition for use according to the invention, wherein the warts are selected among the group consisting of common wart (Verruca vulgaris), flat wart (Verruca plana), filiform or digitate wart, plantar wart (Verruca Pedis), mosaic wart, genital wart (Veneral wart, Condyloma acuminatum, Verruca acuminate), and periungal wart.

According to an embodiment of the invention the warts are common warts.

According to an embodiment of the invention the warts are plantar or flat warts.

According to an embodiment of the invention the warts are filiform or digitate warts.

According to an embodimentof the invention the warts are caused by human papillomavirus (HPV), such as a genotype of HPV causing warts selected among the group consisting of verruca vulgaris, verruca plantaris, verruca plana, and condyloma acuminatum.

According to an embodimentof the invention the warts are caused by human papillomavirus (HPV), such as a type of HPV selected among the group consisting of the types 1, 2, 3, 4, 5, 6, 7, 8, 9, 10, 11, 12, 13, 14, 16, 18, 26, 27, 28, 29, 31, 32, 33, 35, 39, 40, 42, 43, 44, 45, 49, 51, 52, 53, 54, 56, 57, 58, 59, 61, 63, 66, 68, 72, 73, 81, 82, and 89.

According to an embodiment of the invention, the composition may be applied directly on the warts without puncturing the warts or scraping skin of the warts.

According to an embodiment of the invention, the composition may be applied with a soft pen or pad.

According to an embodiment of the invention the total amount (w/w) of formic acid in the composition is 2 - 90%, more preferred 3 - 85%, preferably 5 - 80%, more preferred 10 - 70%, preferably 20 - 60%, more preferred 30 - 50%, preferably 35 - 40%.

According to an embodiment of the invention the total amount (w/w) of formic acid in the composition is 60 - 90%, more preferred 70 - 90%, preferably 80 - 85%. Such a composition is particularly preferred for use on hard warts. Weekly application is particularly preferred for such a composition.

According to an embodiment of the invention the composition may be used on the palm or the foot sole.

According to an embodiment of the invention the warts are selected among the group consisting of common wart (Verruca vulgaris), plantar wart (Verruca Pedis), and mosaic wart.

Alternatively, according to an embodiment the invention the total amount (w/w) of formic acid in the composition is 10 - 60%, more preferred 15 - 50%, preferably 20 - 40%, more preferred 30 - 35%. Lower percentage of formic acid improves the smell of the composition and tends to increase patient compliance. This composition is particularly preferred for use on soft warts. Daily application is particularly preferred for this composition.

According to an embodiment of the invention the compositionmay be used on other sites than the palm or the foot sole.

According to an embodiment of the invention the composition may be used on the back of the foot, the back of the hand, the face, neck, wrists, knees or genitalia.

According to an embodiment of the invention the warts are selected among the group consisting of flat wart (Verruca plana), filiform or digitate wart, genital wart (Veneral wart, Condyloma acuminatum, Verruca acuminate), and periungal wart.

According to an embodiment of the invention the total amount (w/w) of alkyl ester, such as ethyl lactate, in the composition is 10 - 70%, preferably 15 - 65%, more preferably 20 - 60%, more preferred 30 - 50%, preferably 35 - 40%.

According to an embodiment of the invention the total amount (w/w) of said C1-C4 alkyl ester, such as ethyl lactate, in the composition is 65 - 70%.

According to an embodiment of the invention the total amount (w/w) of said C1-C4 alkyl ester, such as ethyl lactate, in the composition is 10 - 30%, preferably 15 - 20%.

According to an embodiment of the invention the composition comprises lactic acid, preferably wherein the total amount (w/w) of lactic acid in the composition is 2 - 90%, more preferred 3 - 85%, preferably 5 - 80%, more preferred 10 - 70%, preferably 15 -65%, more preferably 20 - 60%, more preferred 30 - 50%, preferably 35 - 40%.

According to an embodiment of the invention the total combined amount of formic acid and C1-C4 alkyl ester, such as ethyl lactate, in the composition is at least 60%, more preferred at least 70%, preferably at least 80%, more preferred at least 90%, preferably at least 95%, more preferably at least 97%, more preferred at least 98%, preferably at least 99%, more preferred 100% (w/w).

According to a further embodiment of the present invention, pharmaceutically acceptable carriers, such as water, oil, glycerol, alcohol or mixtures thereof, can be included in a composition according to the invention, e.g. to achieve a further softening effect on and around the warts.

According to an embodiment, a composition for use according to the invention may comprise additional active ingredients or excipients. Examples comprise, but are not limited to, Callitris Intratropica, Lavandula Angustifolia, Melaleuca Alternifolia, lemon oil, and mixtures of any of these. The person skilled in the art is familiar with additional pharmaceutically acceptable excipients which may be combined with the present invention.

According to an embodiment, a composition for use according to the invention may be made substantially free of water and/or ethanol.

According to an embodiment, the invention concerns a composition for use in a method for topical treatment of warts comprising formic acid as an active ingredient, and less than 15%, preferably less than 10%, more preferred less than 5%, preferably less than 3%, more preferred substantially no water and/or alcohol such as ethanol.

According to an embodiment, the invention concerns a composition for use in a method for topical treatment of warts comprising formic acid as an active ingredient, such as a composition according to the invention, and further comprising at least one colorant, e.g. a dye or a pigment, such as carotene. This embodiment may facilitate correct application on warts, and avoid application on surrounding tissue and skin. Particularly preferred is a colorant with a discrete color, or a color, which vanishes from the warts after application. This may improve patient compliance.

According to an embodiment, a composition for use according to the invention may be in the form of an emulsion, cream, paste, ointment, lotion, suspension, gel, spray, and/or together with topical carriers suitable for the treatment.

According to an embodiment, a composition for use according to the invention may be for human use or for veterinary use.

According to an embodimentof the invention the composition may comprise formic acid and at least one C1-C4 alkyl ester of lactic acid, malic acid, tartaric acid, citric acid, or a mixture of any of these, preferably ethyl lactate; preferably for the treatment of warts.

According to an embodiment, the invention concerns use of formic acid and at least one C1-C4 alkyl ester of lactic acid, for the manufacture of a composition for the topical treatment of warts, wherein the total amount (w/w) of said ester in the composition is 10 - 70%.

All proportions and percentages are in weight/weight unless otherwise mentioned.

All cited references are incorporated by reference.

The accompanying Examples are provided to explain rather than limit the present invention. It will be clear to the person skilled in the art that aspects, embodiments and claims of the present invention may be combined.

### Examples

### Example I

Three different compositions were compared by topical application on warts on human subjects.

A first composition (A) was prepared by mixing 30% formic acid with 70% ethyl lactate (w/w).

A second composition (B) was prepared by mixing 85% formic acid with 15% ethyl lactate (w/w).

The third composition (C) was GSK Duofilm, indicated to comprise 16.7% salicylic acid and 16.7% lactic acid.
90 patients with warts were divided into three groups of 30 patients. The groups received treatment as follows:
Group I: Composition (A). The composition was applied once daily for a maximum of 12 weeks or until the warts were removed.
Group II: Composition (B). The composition was applied once weekly for a maximum of 12 weeks or until the warts were removed.
Group III: Composition (C). The composition was applied once daily for a maximum of 12 weeks or until the warts were removed.

| Number of patients | Group I | Group II | Group III |
|---|---|---|---|
| Dropped out | 6 | 7 | 8 |
| Decreased | 3 | 1 | 2 |
| Failure | 3 | 2 | 5 |
| Resolved | 18 | 20 | 15 |
| Sum | 30 | 30 | 30 |

| Patients | Group I | Group II | Group III |
|---|---|---|---|
| Dropped out | 20% | 23% | 27% |

| Among non-dropped out patients | Group I | Group II | Group III |
|---|---|---|---|
| Decreased | 13% | 4% | 9% |
| Failure | 13% | 9% | 23% |
| Resolved | 75% | 87% | 68% |

| Number of warts | Group I | Group II | Group III |
|---|---|---|---|
| Resolution | 50% | 90% | 45% |

| | | | |
|---|---|---|---|
| "Dropped out" refers to the patients, who did not complete the study, i.e. failed to show up until completion of the study. | | | |

In general, higher formic acid concentration led to higher efficacy. However, complaints were also increasing due to pain upon application of the compositions with higher formic acid contents. It was found that composition (A) was especially suitable for treating warts on other places than the feet and hands, such as other places than the palm and the foot sole, while composition (B) was especially suitable for treating warts on feet and hands, particularly on the palm and the foot sole. Of the three tested compositions, composition (C) led to the highest drop-out rate, the highest failure rate among the patients, and the lowest percentage of resolved warts.

### Comparison Example I

A mixture comprising 85% formic acid and 15% water was applied on warts, using a puncture technique.

The results using this puncture technique was comparable to the effect obtained by using the second mixture (B) according to the invention. However, the use of the second mixture (B) did not necessitate the use of a puncture technique, as the second composition (B) was applied directly on the warts.

### Comparison Example II

A mixture comprising 85% formic acid and 15% water was applied on the skin of a human male. The mixture was foul smelling and painful upon application.

### Contemplated Compositions of the Invention

The compositions of Table I have been made or are contemplated. The compositions may be manufactured by mixing the ingredients at room temperature.

The compositions may be applied on a wart as a drop, with a cotton stick or with a pen comprising the composition.

**Table I**

| Composition No. | Formic acid | Ethyl lactate | Lactic acid | Lavender Oil | Callitris intratropica oil | Melaleuca alternifolia oil | Aqua | Glycerol | **Total** |
|---|---|---|---|---|---|---|---|---|---|
| 1 | 85% | 15% | | | | | | | **100%** |
| 2 | 33% | 56% | 10% | 1% | | | | | **100%** |
| 3 | 3% | 87% | 10% | | | | | | **100%** |
| 4 | 10% | 80% | 10% | | | | | | **100%** |
| 5 | 50% | 30% | 20% | | | | | | **100%** |
| 6 | 50% | 15% | 30% | 1% | 1% | 1% | 1% | 1% | **100%** |
| 7 | 20% | 70% | 10% | | | | | | **100%** |
| 8 | 40% | 35% | 20% | 4% | | | | 1% | **100%** |
| 9 | 35% | 40% | 5% | 10% | 1% | 4% | | 5% | **100%** |
| 10 | 60% | 20% | 5% | | | | 10% | 5% | **100%** |
| 11 | 20% | 30% | 10% | 15% | | | 10% | 15% | **100%** |
| 12 | 80% | 15% | | 3% | | | | 2% | **100%** |
| 13 | 75% | 5% | 15% | | 5% | | | | **100%** |
| 14 | 70% | 25% | | 2% | | 2% | | 1% | **100%** |
| 15 | 30% | 25% | 40% | | | | | 5% | **100%** |
| 16 | 45% | 15% | 40% | | | | | | **100%** |
| 17 | 10% | 90% | | | | | | | **100%** |
| 18 | 15% | 80% | 5% | | | | | | **100%** |
| 19 | 35% | 50% | 10% | | | | 5% | | **100%** |
| 20 | 30% | 15% | 50% | 1% | 1% | 1% | 1% | 1% | **100%** |
| 21 | 40% | 50% | 10% | | | | | | **100%** |
| 22 | 25% | 50% | 25% | | | | | | **100%** |
| 23 | 14% | 70% | 10% | 1% | | | | 5% | **100%** |
| 24 | 35% | 65% | | | | | | | **100%** |
| 25 | 30% | 70% | | | | | | | **100%** |

### Measurement of Contact Angle

Measurements of the contact angle of mixtures of a) formic acid and water; and b) formic acid and ethyl lactate were conducted according to the following protocol:
1) Coverslip cleaned with methanol were washed with H₂O and dried with N₂.
2) The liquid mixtures were mixed on a rotation table for 10 min.
3) Using 2 coverslips for each comparison, three droplets of each of the samples are added onto each of the two coverslips.

Each solution (mixture) was measured on two distinct surfaces (3 drops on each surface). From the contact angle studies a significant difference between the samples was observed. The results are provided in Table II below.

**Table II**

| Average Measured Contact Angles of Mixtures | + water to 100% (w/w) | +ethyl lactate to 100% (w/w) |
|---|---|---|
| 35% formic acid | 31 | 16 |
| 85% formic acid | 18 | 10 |

### Conclusions

Preliminary experiments indicate selected compositions according to the invention may in favorable cases completely eradicate a treated wart within 2 - 3 days upon daily topical administration. Other cases may require longer time, such as 1, 2 or 3 weeks. Particular success has been achieved with filiform warts in the face of a patient. In contrast, the mixture of the Comparison Example II took several weeks, and as an average about a month, before the warts were removed from the hands and feet of the patients. No success with the removal of warts in the face of a patient was reported with the mixture of the Comparison Example. Further, some subjects have complained about pain upon application of the Comparison Example II, e.g. if this mixture was applied on skin.

Lower concentrations of formic acid tend to remove the smell and the pain associated with application of a wart mixture comprising formic acid. Earlier, it has been conventional wisdom that formic acid should be present in a high concentration, such as about 85%, to be effective against warts. The present invention appears to allow the treatment of warts using lower concentrations of formic acid. Lower concentrations allow more frequent, such as daily, application of the composition, and thus the concentration of active ingredients *in situ* may be kept approximately constant as compared to the situation where a composition is applied on a weekly basis. High concentration of formic acid is associated with the disadvantage of pain and creation of wounds upon accidental application on the skin surrounding a wart. Thus, high concentration of formic acid usually require longer intervals between application, as the skin needs time to reconstitute between treatments.

Further, no pretreatment with hot water or scraping of hard skin appeared necessary. In addition, as the smell was not repulsive as opposed to the Comparison Example, a composition of the present invention could readily be applied to the face, arms, legs and body of the patient with good compliance. A composition according to the invention could be applied to other parts of the skin, such as the hands and feet of the patients, with similar good compliance.

## Claims

1. Composition comprising formic acid and at least one C1-C4 alkyl ester of lactic acid, for use in a method for topical treatment of warts wherein the total amount (w/w) of said ester, in the composition is 10 - 70%.

2. Composition for use according to claim 1, wherein said at least one C1-C4 alkyl ester is ethyl lactate.

3. Composition for use according to any of the preceding claims, for application directly on the warts without puncturing the warts or scraping skin of the warts.

4. Composition for use according to any ofthe preceding claims, wherein the total amount (w/w) of formic acid in the composition is 1- 90%, more preferred 3 - 85%, preferably 5 - 80%, more preferred 10 - 70%, preferably 15- 65%, more preferably 20 - 60%, more preferred 25 - 50%, preferably 30 -40%, more preferred 30 - 35%.

5. Composition for use according to any ofthe preceding claims, wherein the total amount (w/w) of formic acid in the composition is 60 - 90%, more preferred70- 90%, preferably 80 - 85%.

6. Composition for use according to claim 5, wherein said composition is for use on hard warts.

7. Composition for use according to claim 5 or 6, wherein said composition is for use on the palm or the foot sole.

8. Composition for use according to any of the claims 1-4, wherein the total amount (w/w) of formic acid in the composition is 10 - 60%, more preferred 15 - 50%, preferably 20 - 40%, more preferred 30 - 35%.

9. Composition for use according to claim 8, for use on soft warts.

10. Composition for use according to claim 8 or 9, for use on other sites than the palm or the foot sole.

11. Composition for use according to any of the claims 8 - 10, for use on the back of the foot, the back of the hand, the face, neck, wrists, knees or genitalia.

12. Composition for use according to any ofthe preceding claims, comprising about 30% formic acid and about 70% of ethyl lactate.

13. Composition for use according to any of the preceding claims, comprising about 85% formic acid and about 15% of ethyl lactate.

14. Pen for topical administration, comprising a composition for use according to any of the preceding claims.

15. Use of formic acid and at least one C1-C4 alkyl ester of lactic acid for the manufacture of a composition for the topical treatment of warts, wherein the total amount (w/w) of said ester in the composition is 10 - 70%.

## Patentansprüche

1. Zusammensetzung, die Ameisensäure und mindestens einen C1-C4-Alkylester von Milchsäure umfasst, für die Verwendung in einem Verfahren für die topische Behandlung von Warzen, wobei die Gesamtmenge (m/m) des Esters in der Zusammensetzung 10-70 % beträgt.

2. Zusammensetzung für die Verwendung nach Anspruch 1, wobei der mindestens eine C1-C4-Alkylester Ethyllactat ist.

3. Zusammensetzung für die Verwendung nach einem der vorstehenden Ansprüche für die direkte Applikation auf den Warzen ohne Punktion der Warzen oder Abschabung von Haut der Warzen.

4. Zusammensetzung für die Verwendung nach einem der vorstehenden Ansprüche, wobei die Gesamtmenge (m/m) an Ameisensäure in der Zusammensetzung 1-90 %, bevorzugter 3-85 %, bevorzugt 5-80 %, bevorzugter 10-70 %, bevorzugt 15-65 %, bevorzugter 20-60 %, bevorzugter 25-50 %, bevorzugt 30-40 %, bevorzugter 30-35 % beträgt.

5. Zusammensetzung für die Verwendung nach einem der vorstehenden Ansprüche, wobei die Gesamtmenge (m/m) an Ameisensäure in der Zusammensetzung 60-90 %, bevorzugter 70-90 %, bevorzugt 80-85 % beträgt.

6. Zusammensetzung für die Verwendung nach Anspruch 5, wobei die Zusammensetzung für die Verwendung auf harten Warzen vorgesehen ist.

7. Zusammensetzung für die Verwendung nach Anspruch 5 oder 6, wobei die Zusammensetzung für die Verwendung auf der Handfläche oder der Fußsohle vorgesehen ist.

8. Zusammensetzung für die Verwendung nach einem der Ansprüche 1-4, wobei die Gesamtmenge (m/m) an Ameisensäure in der Zusammensetzung 10-60 %, bevorzugter 15-50 %, bevorzugt 20-40 %, bevorzugter 30-35 % beträgt.

9. Zusammensetzung für die Verwendung nach Anspruch 8 für die Verwendung auf weichen Warzen.

10. Zusammensetzung für die Verwendung nach Anspruch 8 oder 9 für die Verwendung an anderen Stellen als der Handfläche oder der Fußsohle.

11. Zusammensetzung für die Verwendung nach einem der Ansprüche 8-10 für die Verwendung auf dem Fußrücken, dem Handrücken, dem Gesicht, Hals, den Handgelenken, Knien oder Genitalien.

12. Zusammensetzung für die Verwendung nach einem der vorstehenden Ansprüche, die etwa 30 % Ameisensäure und etwa 70 % Ethyllactat umfasst.

13. Zusammensetzung für die Verwendung nach einem der vorstehenden Ansprüche, die etwa 85 % Ameisensäure und etwa 15 % Ethyllactat umfasst.

14. Pen für die topische Verabreichung, der eine Zusammensetzung für die Verwendung nach einem der vorstehenden Ansprüche umfasst.

15. Verwendung von Ameisensäure und mindestens einem C1-C4-Alkylester von Milchsäure für die Herstellung einer Zusammensetzung für die topische Behandlung von Warzen, wobei die Gesamtmenge (m/m) des Esters in der Zusammensetzung 10-70 % beträgt.

## Revendications

1. Composition comprenant de l'acide formique et au moins un C₁-C₄-alkylester de l'acide lactique, pour une utilisation dans une méthode destinée au traitement topique de verrues, dans laquelle la quantité totale (p/p) dudit ester dans la composition est de 10-70%.

2. Composition pour une utilisation selon la revendication 1, dans laquelle ledit au moins un C₁-C₄-alkylester est le lactate d'éthyle.

3. Composition pour une utilisation selon l'une quelconque des revendications précédentes, destinée à une application directement sur les verrues sans ponctionner les verrues ni gratter la peau des verrues.

4. Composition pour une utilisation selon l'une quelconque des revendications précédentes, dans laquelle la quantité totale (p/p) d'acide formique dans la composition est de 1-90%, plus préférablement 3-85%, préférablement 5-80%, plus préférablement 10-70%, préférablement 15-65%, plus préférablement 20-60%, plus préférablement 25-50%, préférablement 30-40%, plus préférablement 30-35%.

5. Composition pour une utilisation selon l'une quelconque des revendications précédentes, dans laquelle la quantité totale (p/p) d'acide formique dans la composition est de 60-90%, plus préférablement 70-90%, préférablement 80-85%.

6. Composition pour une utilisation selon la revendication 5, où ladite composition est destinée à une utilisation sur des verrues dures.

7. Composition pour une utilisation selon la revendication 5 ou 6, où ladite composition est destinée à une utilisation sur la paume ou sur la plante du pied.

8. Composition pour une utilisation selon l'une quelconque des revendications 1-4, dans laquelle la quantité totale (p/p) d'acide formique dans la composition est de 10-60%, plus préférablement 15-50%, préférablement 20-40%, plus préférablement 30-35%.

9. Composition pour une utilisation selon la revendication 8, destinée à une utilisation sur des verrues molles.

10. Composition pour une utilisation selon la revendication 8 ou 9, destinée à une utilisation sur d'autres sites que la paume ou la plante du pied.

11. Composition pour une utilisation selon l'une quelconque des revendications 8-10, destinée à une utilisation sur le dessus du pied, le dos de la main, le visage, le cou, les poignets, les genoux ou les organes génitaux.

12. Composition pour une utilisation selon l'une quelconque des revendications précédentes, comprenant environ 30% d'acide formique et environ 70% de lactate d'éthyle.

13. Composition pour une utilisation selon l'une quelconque des revendications précédentes, comprenant environ 85% d'acide formique et environ 15% de lactate d'éthyle.

14. Crayon destiné à une administration topique, comprenant une composition destinée à une utilisation selon l'une quelconque des revendications précédentes.

15. Utilisation d'acide formique et d'au moins un C₁-C₄-alkylester de l'acide lactique, pour la fabrication d'une composition destinée au traitement topique de verrues, dans laquelle la quantité totale (p/p) dudit ester dans la composition est de 10-70%.
